# EUROPEAN PATENT SPECIFICATION

(11) **EP 0 815 879 B1**
(45) Date of publication and mention of the grant of the patent: **16.01.2008**
(21) Application number: 97304606.3
(22) Date of filing: 27.06.1997
(51) Int. Cl.: A61L 31/00

(54) **Bioabsorbable medical devices from oxidized polysaccharides**
Bioresorbierbare medizinische Vorrichtungen aus oxidierten Polysacchariden
Dispositifs médicaux biorésorbables en polysaccharides oxidés

(30) Priority: 28.06.1996 US 20758 P
(43) Date of publication of application: 07.01.1998
(73) Proprietor: JOHNSON & JOHNSON MEDICAL, INC., Arlington, Texas 76004-3030 (US)
(72) Inventor: Wiseman, David M., Dallas, TX 75248 (US); Saferstein, Lowell, Edison, NJ 08820 (US); Wolf, Stephen, Neshanic Station, NJ 08853 (US)
(74) Representative: Fisher, Adrian John

(56) References cited:
- EP-A- 0 303 496
- EP-A- 0 492 990
- GB-A- 779 820
- US-A- 4 543 410
- BUDAVARI S.: "The Merck Index, 11th Edition" 1989, MERCK & CO. , RAHWAY, N.J., USA

## Description

### Background

### Field of the Invention

The present invention relates generally to oxidized polysaccharides, especially derivatives of cellulose, and methods for their production and use.

### State of the Prior Art

Surgeons face numerous problems during even routine surgery; one of the most frustrating is the formation of adhesions after a surgical procedure. An adhesion is a connection that occurs between two internal body surfaces that are not normally connected Adhesions may occur for a number of reasons that are unrelated to surgery. However since adhesion formation may be regarded as being analogous to that of scar formation, it is not surprising that adhesions occur after surgery. It has been estimated that adhesion formation occurs in about 90% of all surgical procedures, and that about 10% of these cause post surgical problems (Ellis H The causes and prevention of intestinal adhesions. Br J Surg 69:241-243; 1982; Weibel MA, Majno G. Peritoneal adhesions and their relation to abdominal surgery. Am. J. Surg. 126-345-353,1973).

Depending on the anatomical site of adhesion formation different problems may arise. For example, adhesions involving the fallopian tubes may cause infertility. Adhesions involving the intestine may cause an intestinal obstruction. In the thorax following a cardiac procedure, adhesion formation may seriously complicate a redo sternotomy

The typical approach to reducing adhesion formation is to limit trauma to the areas where adhesions form. However, even the most skilled surgeons induce sufficient trauma during many procedures to induce some degree of surgical adhesions This is especially true during more invasive procedures such as open heart surgery. Also, some patients are more prone to form adhesions. At present, there are no approved drugs or devices which effectively reduce adhesion formation after cardiac surgery. However, a cloth of oxidized regenerated cellulose (INTERCEED^{®} available from Johnson & Johnson Medical, Inc.) has been approved by the United States Food and Drug Administration for reducing surgical adhesions in certain pelvic procedures and has been shown to have a general adhesion limiting effect in other surgical procedures.

Also, it has been suggested that certain water soluble gels made from hyaluronic acid or carboxymethylcellulose may be useful in limiting the formation of surgical adhesions. However, both these substances have certain disadvantages. Hyaluronic acid is somewhat difficult to produce. It is either purified from rooster combs, or it is produced by fermentation. Carboxymethylcellulose (CMC) is very inexpensive to produce, but does not degrade in the body. Like other cellulose derivatives such as methylcellulose and hydroxyethylcellulose, its metabolic fate is uncertain and may be sequestered by cells of the reticuloendothelial system (Hueper WC. Macromolecular substances as pathogenic agents. Arch. Pathol. 33:267-290,1942.; Hueper WC. Experimental studies in cardiovascular pathology. XI. Thesaurosis and atheromatosis produced in dogs by the repeated intravenous injections of solutions of sodium cellulose glycollate. Am. J. Pathol. 21:1021-1029,1945; Hueper WC. Experimental studies in cardiovascular pathology. XII. Atheromatosis in dogs following repeated intravenous injections of solutions of hydroxyethylcellulose. Arch. Pathol. 41:130-138,1946).

Carboxymethylcellulose (CMC) is a member of a class of cellulose derivatives that form water soluble gels. Cellulose itself may be rendered bioabsorbable by exposing it to oxidants. This was first discovered in 1936, by W. Kenyon of the Eastman Kodak Research Laboratories, who was carrying out fundamental research on the oxidation of cellulose. Kenyon found that a new type of product could be made by the use of nitrogen dioxide as an oxidizing agent. The material was soluble in alkali and in contrast to the usual friable materials resulting from other methods of oxidation of cellulose, this material maintained its original form and much of its original tensile strength. It was shown that the product was a copolymer of anhydroglucose and anhydroglucuronic acid. This oxidized cellulose material was developed into a bioabsorbable fabric hemostat by Parke Davis and Johnson & Johnson. A good discussion of the process can be found in the following article and patents, all of which are incorporated herein by reference: "Oxidation of Cellulose," by Richard Kenyon, Industrial and Engineering Chemistry, vol 41 (1) 2-8, 1949; US patents 2,232990 issued 1941; 2,298387 issued 1943; 3,364,2000 issued 1968 Ashton et al. U.S. Patent No. 3,364,200, issued January 16, 1968, and the Boardman et al. U.S. Patent No. 5,180,398, issued January 19, 1993 and its foreign equivalents, EP 0,492,990, Japanese Application No. 361083/91

The oxidizing action of numerous oxidants on cellulose has been studied under widely varying conditions of temperature, pH, time of reaction and concentration. The major problem in studying oxidized celluloses is the difficulty of producing materials which are homogeneous in chemical and physical properties. Several of the oxidants employed are apparently not selective as to the particular hydroxyl groups of the anhydroglucose unit in the cellulose molecules which are attacked. Many methods of oxidation are topochemical. When the oxidation is mild, the products usually consist of an oxidized portion and an unchanged residue of unreacted or only slightly modified cellulose. More drastic oxidation produces a larger proportion of oxidized material accompanied by increased degradation. Physical degradation accompanying the oxidation breaks up the cellulose fibers and usually the material is friable and powders easily.

Although the earliest work on preparing oxidized cellulose with nitrogen dioxide goes back to the late 1930's - 1940's, we recently expanded the chemistry so that it is possible to prepare other bioabsorbable polymers from cellulose derivatives and other carbohydrates. We have recently discovered, that cellulose derivatives such as carboxymethylcellulose, water soluble cellulose monoacetate and methyl cellulose among others can also be oxidized with nitrogen dioxide to bioabsorbable materials. (A Czech Patent 118,765 dated June 15, 1966 to Jozef Tamchyna and Frantisek Skoda discloses oxidizing starch with nitrogen tetroxide but there is no mention of bioabsorbabjlity of the oxidized starch. We have also found that carbohydrates with the formula (C₆H₁₀O₅)ₙ such as guar, konjac, starch, and dextrin can also be oxidized with nitrogen dioxide to bioabsorbable polymers.. We have also oxidized dextran, pustulan and cyclodextrin into bioabsorbable polymers. Alginates were also oxidized.

Methyl cellulose and carboxymethylcellulose (CMC) are two of the most widely used water soluble derivatives of cellulose and have applications in the food, cosmetic and pharmaceutical industries. They are made by the reaction of alkali cellulose with methyl chloride or chloroacetic acid respectively. The reaction conditions are usually chosen such that only partial substitution of the three hydroxyl groups on each cellulose monomer are substituted. Thus cellulose derivatives such as methyl cellulose and carboxymethylcellulose are partially substituted derivatives of cellulose. They dissolve in water to form thick, aqueous solutions, the viscosity of which depends upon the concentration of the polymer and its molecular weight. Methylcellulose has been commercialized by several companies, one of which is Dow Chemical Company who sells the product under the name METHOCEL A ^{®} brand products. Carboxymethylcellulose is sold among others by Aqualon under the generic name sodium carboxymethylcellulose.

Neither methyl cellulose, carboxymethylcellulose nor cellulose acetate are bioabsorbable polymers. If a film, powder, sponge, solution or any device made of these polymers were placed within the body cavity, it would dissolve as a high molecular weight water soluble polymer. Although some excretion is possible, some of the polymer would eventually find its way into the walls of blood vessels, renal glomeruli and the cells of the reticuloendothelial system, possibly initiating damage thereto (Wiseman, D.M. Polymers for the Prevention of Surgical Adhesions. In: Polymer Site Specific Pharmacotherapy. Domb, A. (ed). John Wiley, Chichester, 1994 pp 385).

For a polymer to be bioabsorbable it must be broken down in some manner into low molecular weight fragments that can either be metabolized by the liver or excreted through the kidneys. For example, medical devices made from collagen and gelatin when placed within the body are enzymatically broken down into low molecular weight peptides and amino acid fragments which are metabolized by the liver into new proteins or are excreted by the kidneys. Polymers such as polyglycolide for absorbable sutures and polyanhydrides for controlled release of medicaments are sensitive to moisture and are broken down by body fluids to low molecular weight water soluble fragments that either pass out of the body in the urine or are metabolized by the liver. Oxidized cellulose is stable below pH 7, but as the pH approaches and exceeds that of body fluid, the polymer chain is broken down into water soluble low molecular weight oligosaccharides and sugar like moieties which are passed through the kidneys and eliminated into the urine. A good discussion of the pH sensitivity of oxidized cellulose is presented by Alexander Meller in "Holzforschung." Vol. 14, pg. 78-89, 129-139 (1960).

EP 0 492 990 discloses a process for oxidising cellulose comprising reacting the cellulose with a solution of nitrogen dioxide in a perfluorocarbon solvent.

US 4,543,410 discloses absorbent, coherent, flexible structures in the form of fibrous webs and porous sponges comprising water-insoluble, ring oxidised cellulosic bases consisting of water-insoluble cellulose ethers, cellulose mixed ethers, ring oxidised forms of these cellulose, cellulose ether mixed esters and mixtures of the bases.

### Summary of the Invention

We have discovered that cellulose derivatives can be oxidised with nitrogen dioxide into bioabsorbable polymers. The starting material and oxidising process are relatively inexpensive when compared to naturally occurring bioabsorbable animal polymers such as hyaluronic acid and dextran. Further, they are useful in limiting surgical adhesions, and also for hemostasis, for the controlled release of drugs and as wound dressing materials among many medical uses.

In an aspect of the present invention there is provided a process for preparing a bioabsorbable device comprising the steps of dissolving a cellulose derivative selected from the group consisting of methylcellulose, carboxymethylcellulose or cellulose acetate in water and oxidising the cellulose derivative.

The present invention also provides a bioabsorbable device obtainable by the process of claim 1, comprising an oxidized product of a water-soluble cellulose derivative, wherein the cellulose derivative is methylcellulose, carboxymethylcellulose or cellulose acetate.

Preferably, the device is sterile and gamma irradiation is used as the preferred sterilization method. The device may be provided in the form of a film, gel, powder, fibrous mat or sponge among others.

In the case of oxidized methyl cellulose, it is both bioabsorbable and water soluble and may be mixed with a sufficient quantity of water or physiologically acceptable buffer to form a gel. The gel may be impregnated into a bioabsorbable substrate, such as a cloth formed of oxidized regenerated cellulose. The composite material may also be produced by coating a methyl cellulose solution onto a cloth of rayon or cellulose and drying to produce a film on the surface of the fabric or allowing the methyl cellulose solution to impregnate the interstices of the fabric. When dry the composite can be oxidized with nitrogen dioxide gas to produce a completely bioabsorbable fabric with a bioabsorbable gel forming polymer incorporated within its structure.

The bioabsorbable device according to the invention may be used in a process for inhibiting adhesions comprising the step of applying the bioabsorbable device to a site on the body susceptible to adhesions. One preferred method comprises placing the device, most preferably a gel of oxidized methyl cellulose, into the thoracic cavity to inhibit cardiac adhesions. The gel may also be placed in the abdominal cavity, to prevent abdominal adhesions. The gel may also be applied to the body through a lumen in an endoscope.

### Detailed Description

Of the cellulose derivatives, methyl cellulose is perhaps the most exciting in that it converts into a water soluble bioabsorbable polymer when reacted with nitrogen dioxide whereas the other oxidized cellulose derivatives we examined are water insoluble. The oxidized methyl cellulose polymer forms viscous aqueous gels at concentrations >3% which are stable below pH 7 but fall apart above pH 7 to thin watery solutions. The oxidation of methyl cellulose can be carried out in a number of ways, one of which involves exposing a slurry of methyl cellulose powder to a solution of nitrogen dioxide in an inert solvent such as carbon tetrachloride.. The methyl cellulose slurry is exposed to the solution of nitrogen dioxide for 2-48, preferably 4-16 hours. At the end of the time period, the oxidized methyl cellulose powder is filtered away from the solvent and is washed in 90% isopropyl alcohol, 10% water, or 90% acetone, 10% water to remove the excess nitrogen dioxide gas. The oxidized methyl cellulose powder is dried with 100% acetone or 100% isopropyl alcohol to produce a white powder which then is water soluble. Solutions may be filtered to remove any insoluble material. Oxidized methyl cellulose solutions of concentrations between 0.5 and 5% can be cast onto glass with a casting blade and allowed to dry into films or the aqueous solution can be freeze dried into sponges. The oxidized polymer can also be precipitated from aqueous solutions by pouring the solution into acetone or isopropyl alcohol which are non-solvent for the polymer.

The chemistry of oxidation of methyl cellulose is speculated to be similar to that for oxidation of cellulose. A typical structure of methyl cellulose is shown below. The substitution of methyl ether groups on the hydroxyl groups of cellulose can occur on either the primary hydroxyl or the two secondary hydroxyl groups. The figure below shows an ideal structure with the methyl substitution only on the primary group but in fact METHOCEL A^{®} has a degree of substitution of 1.6-1.9 , which means that more than one methyl group is substituted on each ring structure. The distribution of the methyl groups has been reported by Y.Tezuka, K. Imai, M. Oshima and T. Chiba, Macromolecules Vol. 20, pg. 2413-2418, 1987. When nitrogen dioxide comes in contact with the methyl cellulose polymer, oxidation of some of the secondary alcohol groups to ketones occurs along with oxidation of some of the unsubstituted primary alcohol groups to carboxylic acid.. The oxidized methylcellulose polymer can be characterized by its carboxylic acid content which can range from about 3-8 percent.

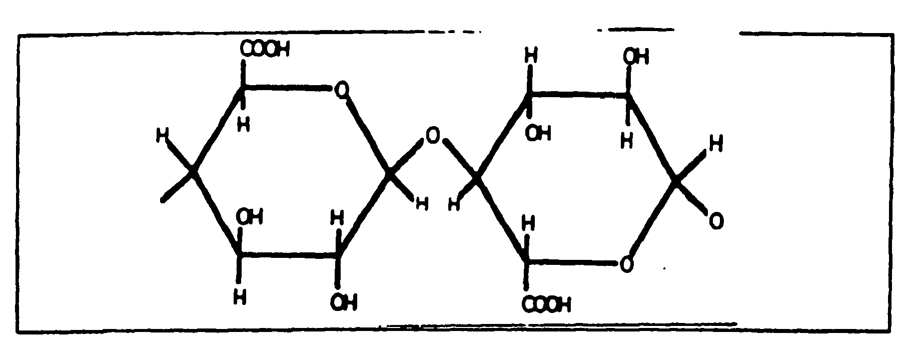

The discovery that a readily available low cost starting material as methyl cellulose can be converted to a bioabsorbable water soluble polymer has major implications in the area of bioabsorbable medical devices. For example recent teachings in the field of post-surgical adhesion prevention show that water soluble gels made from hyaluronic acid (Gel for Preventing Adhesion Between Body Tissues and Process for its Production, WO86/00912, Pharmacia Corp.; (Wiseman, D.M., Johns, D.B. Anatomical synergy between sodium hyaluronate (HA) and INTERCEED^{®} barrier in rabbits with two types of adhesions. (Fertil. Steril. Prog. Suppl. S25, 1993) or viscous solutions of carboxymethylcellulose (Viscoelastic fluid for use in spine and general surgery and other surgery and therapies and method of using same. Pennell PE, Blackmore JM, Allen MD. US Patent 5,156,839 Oct 20, 1992; Assessment of carboxymethylcellulose and 32% dextran 70 for prevention of adhesions in a rabbit uterine horn model. Diamond, MP, DeCherney AH, Linksy, CB, Cunningham T, Constantine B. Int J Fertil 33;278-282, 1988) can be poured into the body cavity to coat tissue and organs with a viscous solution of these polymers. These viscous coatings prevent adjacent tissues from coming in contact with each other for a period of time of from one to ten days, sufficient to allow the tissue to heal and prevent adhesions from forming between the juxtaposed tissue. Viscous aqueous gels of oxidized methyl cellulose can function in the same way. The gel coats organs and tissue to prevent them from coming in contact with each other, then slowly falls apart as the body fluids buffer the solution to above pH 7.

In addition, solutions of oxidized methylcellulose may be used during surgery to minimize tissue damage to abrasion, desiccation and other incidental handling, as has been described for other materials (Goldberg EP and Yaacobi Y, Method for preventing surgical adhesions using a dilute solution of polymer, US Patent 5,080,893, Jan 14 1992, incorporated herein by reference; Diamond, MP and the Sepracoat™ Adhesion Study Group: Precoating with Sepracoat™ (HAL-C™) reduces postoperative de novo adhesion formation in a multicenter randomized, placebo-controlled gynecologic clinical trial. J Soc Gynecol Invest 3;2 Suppl. 90A, 1996).

Sponges or films of oxidized methyl cellulose can be sterilized by gamma irradiation. When placed in the body they will slowly turn into a viscous gel and then gradually dissolve away as the polymer is degraded and solubilized into water soluble fragments. Sponges or films of oxidized methyl cellulose can be used as devices for the controlled release of medicaments by incorporating into the polymer solution a medicament and either casting a film, freeze drying the solution into a sponge, or using the viscous medicated solution as a gel. Oxidized methyl cellulose can also function as a replacement for synovial fluid for the lubrication of a joint, tendon sheath or bursa.

To prepare oxidized methyl cellulose films, methyl cellulose such as METHOCEL A^{®} polymer grades A15LV, A4C, A15C or A4M (available from Dow Chemical) with a degree of substitution of 0.5-1.92 is dissolved in water at 60°C (140° F) according to the manufacturer's instructions, at concentrations from 0.5%w/w to 15% w/w. The solution is cast onto a flat surface at nominal thicknesses from 0.1 mm (5/1000") to 5 mm (200/1000") and allowed to dry. The clear, pliable films are oxidized in either the gas phase with nitrogen dioxide gas or with a solution of nitrogen dioxide in an inert solvent such as carbon tetrachloride or Freon 113. In the preferred gas phase oxidation, the methyl cellulose films are placed in a resin kettle which is flushed with nitrogen gas to displace air. Chilled nitrogen dioxide of 1-3 times the weight of the films is placed in a small flask attached to the resin kettle via a side arm. The nitrogen dioxide gas is allowed to evaporate slowly into the resin kettle and envelop the films. The resin kettle is equipped with a cold condenser vented to the atmosphere which prevents pressure build up. The films of methyl cellulose are exposed to the nitrogen dioxide gas for a period of 2-48 hours, preferably 4-16 hours. At the end of this period the resin kettle is flushed with nitrogen to clear it of excess nitrogen dioxide gas and the films are removed. The oxidized films are washed in 90% isopropanol solution several times to remove adhering oxidant.

The resultant clear, pliable film material is found to dissolve in 0.5N NaOH to give a water thin solution, indicating that it is likely to be bioabsorbable. The oxidized film will also dissolve in water to produce a viscous, acidic, solution. The oxidized methyl cellulose is characterized by its carboxylic acid content which ranges from 3-8%. This oxidized film material was then sterilized by gamma irradiation 18 kJ/kg (1.8 MRad) and implanted subcutaneously in rats. Macroscopic observation of the implant site 10 and 20 days post operative revealed no visible signs of the test material in any of the eight animals implanted with the oxidized methyl cellulose Microscopic evaluation of the cellular response concluded that the test material was rapidly cleared from the subcutaneous implant. At 10 days postoperatively test material had provoked no unusual or unexpected cellular reaction. Sterilized films of oxidized-methyl cellulose were also tested for their ability to prevent pericardial or abdominal adhesions.

The oxidized methyl cellulose film can also be impregnated onto a fabric for example a fabric composed of oxidized regenerated cellulose or an absorbable mesh made from lactide glycolide copolymer.

To prepare an oxidized methyl cellulose gel, methyl cellulose powder such as METHOCEL^{®} A4C, A15-LV or A4M available from Dow Chemical Company with a degree of substitution of 0.5-1.92 is spread evenly in a dish to a depth of 2-8 mm. The dish is placed inside a chamber which is flushed with nitrogen gas to displace the oxygen. Nitrogen tetroxide gas in amounts from one half to 3 times the weight of the methylcellulose powder is introduced into the chamber which is vented to a caustic trap to absorb the oxides of nitrogen. The exposure to nitrogen tetroxide may be carried out from two to forty eight hours. Following exposure the vessel is purged with nitrogen, the oxidized powder is washed with 70-90% isopropyl alcohol and air dried. Alternatively the methylcellulose powder can be suspended in an inert liquid such as Freon 113 in which the nitrogen tetroxide has been dissolved and this suspension stirred for 2-48 hours. The oxidized powder is separated from the inert liquid by filtering off the powder at the end of the reaction time period and washing the powder in 70-90% isopropyl alcohol. In the case of methylcellulose, the oxidized powder is soluble in water which may be cleared of some particulates by dissolving in water at a concentration of between 0.5 and 10% and filtering to remove insolubles. The resulting solution can be used as is or can be lyophilized to produce a sponge for testing.

Carboxymethylcellulose and cellulose mono-acetate are two other water soluble cellulose derivatives that have been converted into bioabsorbable polymers through the action of nitrogen dioxide. Cellulose acetate is water soluble when the degree of substitution of the acetate is below 1 This water soluble polymer from Celanese Corporation should be distinguished from the more common non-water soluble cellulose acetate used to make textile fabrics which has a degree of substitution of2 or higher. Carboxymethylcellulose is produced by Aqualon in several grades with degrees of substitution between 0.38 and 45 and all grades are water soluble. When carboxymethylcellulose or cellulose acetate are oxidized with nitrogen dioxide, the resulting oxidized material is not water soluble but will dissolve in aqueous solutions with a pH> 7 to yield thin, low viscosity solutions, indicating degradation and alkaline sensitivity of the oxidized polymer.

Carboxymethylcellulose and cellulose acetate can be made into films and sponges from dilute solutions of the polymer in water. Casting a dilute aqueous solution of carboxymethylcellulose or cellulose monoacetate on glass or plastic plates with a draw knife produces a film when dry. Freeze drying a dilute aqueous solution of between 0.5%-3% concentration will produce a sponge of these polymers. The films and sponges can be oxidized with nitrogen dioxide gas over a 16 hour period to produce the corresponding oxidized polymer which are now water insoluble and bioabsorbable. Films and sponges of either oxidized carboxymethylcellulose or oxidized cellulose acetate can be sterilized by gamma irradiation. These bioabsorbable devices can make good delivery systems for drugs, medicaments, barriers for adhesion prevention and absorbable hemostats to help arrest bleeding during surgery.

Other cellulose derivatives that may be oxidized include ethyl cellulose. The commercially available ethyl cellulose polymer from Hercules has a DS of 2.46 which is too high to allow sufficient primary and secondary hydroxyl groups on the cellulose backbone to become oxidized, and to be rendered bioabsorbable, as evidenced by dissolution in 0.5N sodium hydroxide. However if ethyl cellulose with a degree of substitution of 0.3 to 1.0 is oxidized with nitrogen tetroxide, it will be transformed into a bioabsorbable oxidized cellulose derivative.

### EXAMPLES

Examples 1-5 show methods of oxidizing cellulose derivatives by exposure to nitrogen dioxide in the gas phase. This oxidation may also be accomplished by exposure to nitrogen dioxide which is dissolved or carried in an appropriate solvent such as PF5060^{®} as was taught by Boardman et al (US patent 5,180,398). To ensure that the product does not dissolve during the washing phase, the amount of water in the washing solvent must be reduced to less than 50%, the remainder of the solvent being comprised of an alcohol such as isopropyl alcohol.

### Example #1.

### Preparation of a Solution of Oxidized Methyl Cellulose

25 grams of METHOCEL^{®} A4M from Dow Chemical Co. was dissolved in 808 grams of distilled water to make a 3% solution. The solution was poured into trays and freeze dried to make sponges. The sponges were cut into small pieces and placed in a resin kettle. The kettle was flushed with nitrogen gas to displace the air for 5 minutes. 15 grams of chilled nitrogen dioxide was placed in a flask attached to the resin kettle by a side arm and the gas was allowed to diffuse into the sponges over a 24 hour period. At the end of this time period the sponges were removed from the resin kettle and allowed to degas. They were then washed 3 times, each with a one liter solution of 90% isopropyl alcohol and 10% water. The sponges were dried in 100% isopropyl alcohol. The dried oxidized sponges, 25 grams, were dissolved in 2475 grams of distilled water and filtered to remove any insolubles. The pH of this solution was 2.3. The clear solution was poured into trays and freeze dried. A 0.5 gram piece of dried sponge was dissolved in 10 ml of 0.5 N NaOH and titrated for percent carboxylic acid. This sample was found to have a 7 weight percent carboxylic acid content.

Ten grams of this sponge was dissolved in 115 grams of water to make an 8% solution. This viscous solution can be used to prevent abdominal and pelvic adhesions.

### Example #2

### Preparation of Oxidized Methyl Cellulose Films.

A 3% aqueous solution of METHOCEL^{®} A4M from Dow Chemical Company was poured onto a glass plate and drawn down with a draw knife set at 0.64 mm (25 mils). The film was allowed to dry overnight and removed the next day from the glass plate to produce a 0.08 mm (3 mil) film. Three grams of the film were placed in a resin kettle which was flushed with nitrogen gas to displace the air. A small flask containing 2 grams of chilled nitrogen dioxide liquid was attached to the head of the resin kettle with a side arm adapter. The nitrogen dioxide was allowed to diffuse into the resin kettle to oxidized the film over a 16 hour period. At the end of this time the film was removed from the resin kettle and washed with a solution of 90% isopropyl alcohol and 10% water to remove any residual gas from the film. This film can be sterilized by gamma irradiation and used as a barrier for adhesion prevention. A 0.5g piece of the film dissolved in 10 ml of a 0.5N sodium hydroxide solution to give a clear solution with low viscosity, indicating its likely bioabsorbability. Another 0.5g piece of film dissolved in 10ml of water to give a solution with moderately high viscosity. This film can be sterilized by gamma irradiation and used as a barrier for adhesion prevention.

### Example #3.

### Preparation of Oxidized Carboxymethylcellulose Sponges.

Ten grams of carboxymethylcellulose grade 7HF with a degree of substitution of 0.65-0.90 from Aqualon was dissolved in 490 grams of water to make a 2% solution. The solution was poured into 3x4 in trays approximately one quarter inch deep. The solutions were frozen on the shelf of a freeze dryer and when fully frozen the vacuum pump was turned on to produce a vacuum of 13 Pa (100 millitore). The shelf temperature was adjusted to 15°C and lyophilization was carried out for 20 hours. At the end of this time period the trays were removed from the freeze dryer and sponges of carboxymethylcellulose were obtained from each tray. These sponges were soft but still water soluble. The sponges, about 10 grams total, were placed in a resin kettle to which a side arm was connected to a small flask. The resin kettle was flushed with nitrogen gas for several minutes then 10 grams of chilled nitrogen dioxide was added to the small flask and slowly allowed to diffuse into the resin kettle containing the carboxymethylcellulose sponges. After 20 hours exposure to the nitrogen dioxide gas, the sponges were removed from the resin kettle and washed with 1 liter of a 50:50 solution of isopropyl alcohol and water. This washing was performed twice. The sponges were then placed in a tray under a source of running water and washed with fresh water for 5 minutes. The sponges were air dried after washing in 1 liter of 100% isopropyl alcohol or they could be freeze dried after washing in water. In freeze drying to make sponges, complete drying is desirable otherwise the sponge once formed may reabsorb moisture from the atmosphere.

The oxidized carboxymethylcellulose sponges are no longer water soluble. One gram however, dissolves in 10 ml of 0.5 N sodium hydroxide to give a clear solution with very low viscosity showing that the oxidized carboxymethylcellulose sponge degrades at the high pH of this sodium hydroxide solution. Titration of the sponge for carboxylic acid groups showed that there were more acid groups present after oxidation then before. The increase in carboxylic acid content due to ring oxidation alone is 6%. The sponges are soft, have fine pore size and will absorb 15 times their weight in water. These sponges can be sterilized with gamma irradiation and after sterilization sponges implanted subcutaneously in rats absorb within ten days with no visible tissue reaction. Oxidized carboxymethylcellulose sponges make good hemostats. When tested on a swine splenic incision model, these sponges will stop bleeding in an average of 2.5 minutes compared to oxidized cellulose fabric (SURGICEL)^{®} which stops bleeding in 6.5 minutes. Sponges of oxidized carboxymethylcellulose can also be used to fill tumor beds, to cover wounds, as diaphragms for contraceptive control and as drug delivery systems. This method is preferred for the production of sponges of oxidized methylcellulose.

### Example #4

### Preparing Films of Oxidized Carboxymethylcellulose

Solutions of carboxymethylcellulose can be cast on glass plate with a draw blade and allowed to dry to give films which also can be oxidized by exposure to nitrogen dioxide gas. Films of oxidized carboxymethylcellulose can be used as absorbable barriers to prevent adhesions.

A 3% aqueous solution of carboxymethylcellulose grade 7MF with a DS of 0.90 from Aqualon was prepared and cast on a glass plate with a draw blade set at 30 mils openings. The film was allowed to air dry overnight. The dry films (3 grams) were placed in a resin kettle which was flushed with nitrogen for 5 minutes to remove the air. A side arm was attached to the resin kettle which connected to a small flask containing 3 grams. of chilled nitrogen dioxide. The liquid nitrogen dioxide was allowed to vaporize and diffuse into the flask containing the films of carboxymethylcellulose. The films were exposed to the gas for a period of 20 hours. At this point they were removed from the resin kettle and washed with water until the pH of the washings were above 3. The films were dried with isopropyl alcohol or acetone. Some were plasticized with 10% glycerol which imparted flexibility to the dry films. The films are water insoluble but dissolve in sodium hydroxide. When titrated for carboxylic acid content they are found to have a 26 weight percent acid content. The increase in carboxyl content from ring oxidation is 7%. The films also absorb within ten days when implanted subcutaneously in rats. These absorbable films make good barriers for the prevention of adhesions.

### Example #5.

### Preparation of Oxidized Cellulose Acetate Devices

A 2% aqueous solution of water soluble cellulose monoacetate available from Celanese Corporation was prepared and poured into trays. The solutions were freeze dried in a lyophilizer for 24 hours White, soft, sponges were prepared by this procedure. The sponges (3 grams) were placed in a resin kettle to which a side arm was connected to a flask. The resin kettle was flushed with nitrogen for 5 minutes. The flask was then filled with 3 grams of chilled nitrogen dioxide and-the liquid was allowed to vaporize and diffuse into the cellulose acetate sponges. After 20 hours exposure to the gas, the sponges were removed and washed with water until the pH of the wash water was above 3.0. The sponges were dried by washing two times with 1 liter of isopropyl alcohol. The sponges are white, soft, and non water soluble. They will dissolve in sodium hydroxide and titration of the sponge for carboxylic acid groups showed a 13.3% carboxylic acid content. The sponges were tested for hemostatic efficacy and were found to stop bleeding on a swine splenic incision model in 3 minutes when averaged over 6 tests compared to oxidized cellulose fabric (SURGICEL)^{®} which stopped bleeding in 6 minutes in this same test.

Films of cellulose acetate can also be prepared by casting an aqueous solution of the polymer onto a glass plate and allowing the solution to dry. Gaseous oxidation of the cellulose acetate films will produce oxidized celluloseacetate films which are no longer water soluble. Oxidized cellulose acetate is a bioabsorbable material and can function as a hemostat, wound dressing, adhesion barrier, controlled release device and in other medical functions wherever a bioabsorbable device is needed.

### Example #6.

### Preparation of a composite oxidized methylcellulose and oxidized cellulose fabric.

A dilute aqueous solution of 1.0% concentration of METHOCEL^{®} A15LV from Dow Chemical Company with a degree of substitution of 1.65 was prepared by dispersing 1.0 grams of methylcellulose powder in 30 grams of water heated to 95°C. Mixing was continued until all the particles were thoroughly wetted. To this dispersion was added 69 grams of cold water and stirring was continued until all the powder had dissolved and viscosity increased.

A five gram piece of a knitted rayon fabric was dipped into the METHOCEL^{®} A15-LV solution for 20 seconds then removed and allowed to dry. The dry impregnated cloth weighed 5-25 grams indicating a 5% pick up of methyl cellulose.

The dry methyl cellulose impregnated fabric was placed in a resin kettle which was flushed with nitrogen gas to displace air and then exposed to 15 grams of nitrogen dioxide gas for a period of 16 hours. At the end of this period the resin kettle was purged of the excess nitrogen dioxide by a nitrogen purge which displaced the oxidant to a caustic trap to neutralize the nitrogen dioxide. The fabric was removed and washed in 300ml of isopropyl alcohol several times to remove adhering oxidant. The fabric was then allowed to air dry. Titration of a sample of the fabric for carboxylic acid content indicated an 18% carboxylic acid content and complete solubility in 0.5N sodium hydroxide. The oxidized composite of oxidized cellulose fabric impregnated with oxidized methylcellulose can be used to prevent postsurgical adhesions.

### Example #7

### Preparation of a composite oxidized carboxymethylcellulose and oxidized cellulose fabric.

A 2.5% aqueous solution of carboxymethylcellulose, grade 9M8 from Aqualon with a degree of substitution of 0.8-0.95 was cast onto a woven rayon fabric and drawn down with a draw blade and allowed to dry on the surface of the woven fabric so that many of the pores of the fabric were bridged by a thin film of carboxymethylcellulose. The dry woven rayon fabric with its thin film of carboxymethylcellulose on the surface (weighing a total Of 10 grams) was placed in a resin kettle equipped with a magnetic stir bar and a condenser vented to a caustic bath to prevent pressure build up. A side arm was attached to the head of the resin kettle which led to a small flask in which was placed 15 grams of cold nitrogen dioxide. The nitrogen dioxide was allowed to slowly evaporate into the resin kettle and envelop the composite of woven rayon and CMC film. After 20 hours of exposure the excess gas was vented out with nitrogen gas and the fabric removed and washed in 500 ml of isopropyl alcohol several times The fabric was allowed to air dry and a small sample was titrated for acid content and found content and found to have 20% carboxylic acid content. The oxidized sample was completely soluble in 0.5N sodium hydroxide. The oxidized CMC film on the surface of the oxidized woven rayon fabric was difficult to delaminate. When implanted subcutaneously in rats, the material was absorbed within 28 days with minimal tissue reaction. The composite can be used to prevent post surgical adhesions.

### Reference Example #8.

### Preparation of powders of oxidized methyl or oxidized carboxymethylcellulose

A slurry of carboxymethylcellulose or methyl cellulose powder is made in an inert solvent such as carbon tetrachloride.. The slurry is exposed to a solution of nitrogen dioxide in the inert solvent for 2-48 preferably 4-16 hours. At the end of the time period, the oxidized methyl or carboxymethylcellulose powder is filtered away from the solvent and is washed in 90% isopropyl alcohol, 10% water, or 90% acetone, 10% water to remove the excess nitrogen dioxide gas. The oxidized methyl or carboxymethylcellulose powder is dried with 100% acetone or 100% isopropyl alcohol to produce a white powder. The oxidized methylcellulose powder will form an aqueous solution which may be filtered to remove any insoluble material. Oxidized methyl cellulose solutions of concentration between 0.5 and 5% can be cast onto glass with a casting blade and allowed to dry into films or the aqueous solution can be freeze dried into sponges. The oxidized methylcellulose polymer can also be precipitated from aqueous solutions by pouring the solution into acetone or isopropyl alcohol which are non-solvents for the polymer. Bioabsorbable glove lubricants, instrument lubricants and mold release agents may be made by this method.

### EXPERIMENTAL METHODS

All animals are assigned blindly and randomly to a treatment group which is revealed to the surgeon only upon completion of the abrasion. All evaluations are made on a blinded basis.

### The rabbit pericardial adhesion model.

Under anesthesia the thorax of white New Zealand rabbits is entered via a midline sternal incision. The pericardium is similarly opened and the anterior surface of the heart abraded forty times using a piece of gauze wrapped around the forefinger. If an absorbable fabric or barrier such as the present invention is to be used, then an elliptical piece with axes 5 cm × 8 cm (2" x 3") approximately, is placed over the anterior surface, of the heart and may be sutured to the pericardium if desired. The thorax is closed in layers.

Twenty three to thirty days later the animals are sacrificed and the adhesions between the anterior surface of the heart and the underside of the sternum evaluated for adhesions. The percentage adhesion involvement of a strip I cm wide and extending from the apex to the base of the anterior cardiac surface is estimated. This strip represents the surface of the heart in intimate contact with the sternum and where adhesions are most likely to occur and cause a problem for the surgeon attempting a reentry to the thorax.

### EXPERIMENTAL RESULTS

### Cardiac Adhesion Model

A film of methyl cellulose (Grade A15C from Dow Chemical) was oxidized in the gas phase by exposure to nitrogen tetroxide gas. After washing and irradiation it was tested in the rabbit cardiac adhesion model by wetting in saline and then pressing onto a sheet of INTERCEED ^{(R)®} (TC7) Absorbable Adhesion Barrier. An elliptical piece of this composite 5 cm × 3 cm (2 x 1" approx) was placed over the anterior surface of the heart. Four weeks later or the adhesions were evaluated and a significant reduction in adhesion formation was noted.

| | Animal No. | Percent adhesions |
|---|---|---|
| Control (no treatment) | | |
| | 217.03 | 100 |
| | 218.33 | 100 |
| | 218.49 | 80 |
| | 218.04 | 90 |
| | | |
| | Mean | 92.5 |
| | SD | 9.57 |
| | N | 4 |

| Oxidized methyl cellulose on INTERCEED ® Barrier | | |
|---|---|---|
| | 217.04 | 50 |
| | 217.02 | 100 |
| | 219.53 | 80 |
| | 218.32 | 15 |
| | 218.03 | 10 |
| | | |
| | Mean | 51 |
| | SD | 39.4 |
| | N | 5 |

Using Student's t-test, there was a statistically significant reduction in adhesion formation when the oxidized methyl cellulose/INTERCEED^{®} Barrier composite was used (P < 0.05). Historical results from a number of experiments gives an average adhesion score of approximately 80%. oxidized in the gas phase and sterilized by irradiation 18kJ/kg (-1.8. MRad) on dry ice. To 7.5 g of this sterile material were added 115 ml of sterile water and 3 ml of glycine sodium (5% w/v) as a buffer. Unlike the non-oxidized material which dissolves slowly, the oxidized methylcellulose powder dissolved rapidly and easily with shaking for one minute to form a viscous solution.

For comparison a 2% w/w solution of sodium carboxymethylcellulose (CMC) (Hercules, 7H4F) was made using a blender. This is a non-degradable material that forms a solution only after vigorous agitation carboxymethylcellulose was used as a positive control as it has been shown previously to reduce adhesion formation (Viscoelastic fluid for use in spine and general surgery and other surgery and therapies and method of using same. Pennell PE, Blackmore JM, Allen MD. US Patent 5,156,839 Oct 20, 1992 ; Diamond. M.P., DeChemey, A.H., Linsky, C.B., Cunningham, T., Constantine, B., Assessment of carboxymethylcellulose and 32% dextran 70 for prevention of adhesions in a rabbit uterine horn model. Int.J.Fert. 33;278-282, 1988).
The carboxymethylcellulose and oxidized methylcellulose solutions were of comparable viscosity. The standard rabbit uterine horn model was performed. Just prior to closure, 20 ml of either oxidized methylcellulose or carboxymethylcellulose the solutions were instilled into the abdominal cavity. Two weeks later adhesions were evaluated blindly.

The present invention provides many advantages over the prior art. For instance, prior teachings regarding cellulose derivatives appear limited to those which are not degradable by hydrolysis but are probably eliminated from the body intact, or are sequestered by the endothelial system. The materials of the present invention provide similar chemical or physical properties but may degrade into small excretable fragments by simple hydrolysis.

In the form of a film or sheet, these materials may be useful for the prevention of adhesions or as hemostats. The materials may also be formed into sponges, primarily for use as hemostats. However, a sponge of oxidized methylcellulose placed into a specific location within the body, would quickly dissolve into a protective gel. These sponges can also be used to absorb fluid from exuding wounds since they absorb at least times their weight of saline. Films or sponges may be used as a primary wound dressing to protect the wound and to provide a moist environment for healing.

All of these materials possess acidic groups which may render them bactericidal. This is a particular advantage in the reduction of infection. The acidic sites may also make these materials suitable for binding with certain drugs, providing a matrix for their controlled released delivery. For example, growth factors may bind to the acidic groups and are subsequently released into a body cavity by ion exchange or as the polymer degrades.

The viscoelastic nature of these materials makes them useful for a number of applications in addition to adhesion prevention or drug delivery by acidic binding. The viscous nature of solutions or sheets of these materials alone may be useful for drug delivery. The materials could also be useful as a degradable surgical lubricant for instruments and gloves. The use of these degradable materials as a mold release agent for medical devices or as a powder for glove lubrication is a distinct advantage over the use of talc or starch which some glove users prefer to avoid. Powders of oxidized methyl- or carboxymethylcellulose may be prepared by the slurry method described in reference example 8.

The materials provide characteristics similar to other materials which are either more expensive or difficult to characterize because they are derived from natural or fermentation sources. Such materials include hyaluronic acid, heparin, chondroitin sulfate and dextran. Thus the relatively inexpensive and easy-to-standardize materials of the present invention may be used as blood expanders, and viscoelastic aids in ocular and orthopedic surgery and tissue augmentation materials. The materials produced may be sterilized by irradiation.

Cross-linking and derivitisation methods known in the art may be used to refine the characteristics of the materials described by this invention, or to bind, covalently or otherwise, certain drugs to the materials. The use of crosslinking agents such as dimethylolurea (bis(N-(hydroxymethyl urea)), divinyl sulfone etc. with the oxidized cellulose derivatives will slow down their bioabsorbtion and will allow them to last in the body longer. In some cases this attribute may enhance their performance. A lightly crosslinked gel of oxidized methyl cellulose will have higher gel strength and be somewhat firmer than non-crosslinked gels.

## Claims

1. A process for preparing a bioabsorbable device comprising the steps of dissolving a cellulose derivative selected from the group consisting of methylcellulose, carboxymethylcellulose or cellulose acetate in water and oxidising the cellulose derivative.

2. The process of claim 1, wherein the cellulose derivative is:
carboxymethylcellulose with a degree of substitution between 0.38 and 1.45; or
methylcellulose with a degree of substitution between 0.5 and 1.92.

3. The process of claim 1 or claim 2, which produces a cellulose derivative having a carboxylic acid content, due to oxidation of primary alcohol groups in the cellulose derivative, of 3 to 12% by weight.

4. The process of any of claims 1 to 3, wherein the oxidizing step comprises placing the polysaccharide into contact with nitrogen dioxide or nitrogen tetroxide.

5. The process of any of claims 1 to 4, further comprising the step of washing the oxidized material in a solvent for the oxidizing agent but a non-solvent for the oxidized material.

6. The process of claim 5, wherein the solvent is isopropyl alcohol and water.

7. The process of any of claims 1 to 6, wherein the cellulose derivative is first impregnated onto a cellulose fabric precursor of a substrate and the cellulose derivative and the precursor are then oxidized together.

8. The process of claim 7, wherein the substrate precursor comprises cellulose.

9. A bioabsorbable device obtainable by the process of claim 1, comprising an oxidized product of a water-soluble cellulose derivative, wherein the cellulose derivative is methylcellulose, carboxymethylcellulose or cellulose acetate.

10. The bioabsorbable device of claim 9, wherein the cellulose derivative is carboxymethylcellulose with a degree of substitution between 0.38 and 1.45;

11. The bioabsorbable device of claim 9 or claim 10, which produces a cellulose derivative having a carboxylic acid content, due to oxidation of primary alcohol groups in the cellulose derivative, of 3 to 12% by weight.

12. The bioabsorbable device of any of claims 9 to 11 which comprises a sufficient quantity of water or a physiologically acceptable buffer to form a gel.

13. A cloth formed of a second bioabsorbable material impregnated with the gel of claim 12.

14. The impregnated cloth of claim 13, wherein the second bioabsorbable material is oxidized cellulose, polylactide-co-glycolide, polydioxanone, polycaprolactone, polyanhydride, polylactide, polyglycolide, gelatin, collagen, elastin, polyphosphazene, hyaluronic acid, polyorthoester, or a combination thereof.

15. The bioabsorbable device of any of claims 10 to 12 in the form of a film or a freeze dried sponge.

16. The bioabsorbable device of claim 15 in the form of a film plasticised with a polyhydroxy alcohol such as glycerol or propylene glycol to impart flexibility.

17. The bioabsorbable device of any of claims 9 to 12, 15 or 16 or the cloth of claims 13 or 14, in sterile form.

18. The bioabsorbable material of any of claims 9 to 12 or 15 to 17 or the cloth of claims 13 or 14 for use in inhibiting adhesions.

## Patentansprüche

1. Verfahren zum Herstellen einer bioabsorbierbaren Einheit, mit den Schritten des Lösens eines Cellulosederivats, das aus der Gruppe bestehend aus Methylcellulose, Carboxymethylcellulose oder Celluloseacetat ausgewählt ist, in Wasser und Oxidieren des Cellulosederivats.

2. Verfahren nach Anspruch 1, bei dem das Cellulosederivat
Carboxymethylcellulose mit einem Substitutionsgrad zwischen 0.38 und 1.45; oder
Methylcellulose mit einem Substitutionsgrad zwischen 0.5 und 1.92 ist.

3. Verfahren nach Anspruch 1 oder Anspruch 2, welches ein Cellulosederivat erzeugt, das einen Carboxylsäuregehalt, aufgrund der Oxidation primärer Alkoholgruppen in dem Cellulosederivat, von 3 bis 12 Gew.% hat.

4. Verfahren nach einem der Ansprüche 1 bis 3, bei dem der Schritt des Oxidierens das Bringen des Polysaccharids in Kontakt mit Stickstoffdioxid oder Stickstofftetroxid aufweist.

5. Verfahren nach einem der Ansprüche 1 bis 4, weiter mit dem Schritt des Waschens des oxidierten Materials in einem Lösemittel für das Oxidiermittel, jedoch einem Nicht-Lösemittel für das oxidierte Material.

6. Verfahren nach Anspruch 5, bei dem das Lösemittel Isopropylalkohol und Wasser ist.

7. Verfahren nach einem der Ansprüche 1 bis 6, bei dem das Cellulosederivat zunächst auf einen Cellulosetextil-Vorläufer eines Substrats imprägniert wird und das Cellulosederivat und der Vorläufer dann zusammen oxidiert werden.

8. Verfahren nach Anspruch 7, bei dem der Vorläufer des Substrats Cellulose aufweist.

9. Bioabsorbierbare Einheit, die durch den Prozeß nach Anspruch 1 erhalten werden kann, welche ein oxidiertes Produkt eines wasserlöslichen Cellulosederivats aufweist, wobei das Cellulosederivat Methylcellulose, Carboxymethylcellulose oder Celluloseacetat ist.

10. Bioabsorbierbare Einheit nach Anspruch 9, bei der das Cellulosederivat Carboxymethylcellulose mit einem Substitutionsgrad zwischen 0.38 und 1.45 ist.

11. Bioabsorbierbare Einheit nach Anspruch 9 oder Anspruch 10, welche ein Cellulosederivat mit einem Carboxylsäuregehalt, aufgrund der Oxidation primärer Alkoholgruppen in dem Cellulosederivat, von 3 bis 12 Gew.% erzeugt.

12. Bioabsorbierbare Einheit nach einem der Ansprüche 9 bis 11, welche eine ausreichende Menge an Wasser oder einem physiologisch akzeptablen Puffer aufweist, um ein Gel zu bilden.

13. Tuch, gebildet aus einem zweiten bioabsorbierbaren Material, das mit dem Gel nach Anspruch 12 imprägniert ist.

14. Imprägniertes Tuch nach Anspruch 13, bei dem das zweite bioabsorbierbare Material oxidierte Cellulose, Polylactidcoglycolid, Polydioxanon, Polycaprolacton, Polyanhydrid, Polylactid, Polyglycolid, Gelatine, Collagen, Elastin, Polyphospan, Hyaluronsäure, Polyorthoester oder eine Kombination aus diesen ist.

15. Bioabsorbierbare Einheit nach einem der Ansprüche 10 bis 12 in der Form eines Filmes oder eines gefriergetrockneten Schwammes.

16. Bioabsorbierbare Einheit nach Anspruch 15 in der Form eines Filmes, der mit Polyhydroxyalkohol, so wie Glycerol oder Propylenglycol, plastiziert ist, um ihm Flexibilität aufzuerlegen.

17. Bioabsorbierbare Einheit nach einem der Ansprüche 9 bis 12, 15 oder 16 oder Tuch nach Anspruch 13 oder 14 in steriler Form.

18. Bioabsorbierbares Material nach einem der Ansprüche 9 bis 12 oder 15 bis 17 oder Tuch nach den Ansprüchen 13 oder 14 zur Verwendung beim Verhindern von Anhaftungen.

## Revendications

1. Procédé de préparation d'un dispositif bioabsorbable qui comprend les étapes consistant à dissoudre un dérivé de cellulose choisi dans le groupe constitué par la méthylcellulose, la carboxyméthylcellulose ou l'acétate de cellulose dans l'eau et à oxyder le dérivé de cellulose.

2. Procédé selon la revendication 1, dans lequel le dérivé de cellulose est :
la carboxyméthylcellulose avec un degré de substitution compris entre 0,38 et 1,45 ; ou
la méthylcellulose avec un degré de substitution compris entre 0,5 et 1,92.

3. Procédé selon la revendication 1 ou la revendication 2, qui produit un dérivé de cellulose ayant une teneur en acide carboxylique, en raison de l'oxydation des groupes alcool primaire dans le dérivé de cellulose, de 3 à 12 % en poids.

4. Procédé selon l'une quelconque des revendications 1 à 3, dans lequel l'étape d'oxydation comprend la mise en contact du polysaccharide avec du dioxyde d'azote ou du tétroxyde d'azote.

5. Procédé selon l'une quelconque des revendications 1 à 4, comprenant en outre l'étape consistant à laver le matériau oxydé dans un solvant pour l'agent d'oxydation mais un non solvant pour le matériau oxydé.

6. Procédé selon la revendication 5, dans lequel le solvant est l'alcool isopropylique et l'eau.

7. Procédé selon l'une quelconque des revendications 1 à 6, dans lequel le dérivé de cellulose est d'abord imprégné sur un précurseur de tissu de cellulose d'un substrat et le dérivé de cellulose et le précurseur sont alors oxydés ensemble.

8. Procédé selon la revendication 7, dans lequel le précurseur de substrat comprend de la cellulose.

9. Dispositif bioabsorbable pouvant être obtenu par le procédé selon la revendication 1, comprenant un produit oxydé d'un dérivé de cellulose soluble dans l'eau, dans lequel le dérivé de cellulose est la méthylcellulose, la carboxyméthylcellulose ou l'acétate de cellulose.

10. Dispositif bioabsorbable selon la revendication 9, dans lequel le dérivé de cellulose est la carboxyméthylcellulose avec un degré de substitution compris entre 0,38 et 1,45.

11. Dispositif bioabsorbable selon la revendication 9 ou la revendication 10, qui produit un dérivé de cellulose ayant une teneur en acide carboxylique, en raison de l'oxydation des groupes alcool primaire dans le dérivé de cellulose, de 3 à 12 % en poids.

12. Dispositif bioabsorbable selon l'une quelconque des revendications 9 à 11, qui comprend une quantité suffisante d'eau ou d'un tampon physiologiquement acceptable pour former un gel.

13. Toile formée par un second matériau bioabsorbable imprégné du gel selon la revendication 12.

14. Toile imprégnée selon la revendication 13, dans laquelle le second matériau bioabsorbable est la cellulose oxydée, un poly lactide-co-glycolide, un polydioxanone, un polycaprolactone, un polyanhydride, un polylactide, un polyglycolide, la gélatine, le collagène, l'élastine, le polyphosphazène, l'acide hyaluronique, un polyorthoester, ou une combinaison de ceux-ci.

15. Dispositif bioabsorbable selon l'une quelconque des revendications 10 à 12, sous la forme d'un film ou d'une éponge lyophilisée.

16. Dispositif bioabsorbable selon la revendication 15, sous la forme d'un film plastifié avec un polyhydroxy alcool tel que le glycérol ou le propylène glycol pour impartir une flexibilité.

17. Dispositif bioabsorbable selon l'une quelconque des revendications 9 à 12, 15 ou 16 ou la toile selon les revendications 13 ou 14, sous une forme stérile.

18. Dispositif bioabsorbable selon l'une quelconque des revendications 9 à 12 ou 15 à 17 ou la toile selon les revendications 13 ou 14, destiné(e) à être utilisé(e) pour inhiber les adhésions.
